# EUROPEAN PATENT SPECIFICATION

(11) **EP 0 839 532 B1**
(45) Date of publication and mention of the grant of the patent: **07.05.2003**
(21) Application number: 97308588.9
(22) Date of filing: 29.10.1997
(51) Int. Cl.: A61K 31/4535, A61P 35/00

(54) **Improvements in or relating to the prophylaxis of breast cancer by the administration of raloxifene**
Verbesserung in oder in Bezug auf der Vorbeugung von Brustkrebs durch Verabreichung von Raloxifen
Améliorations de ou relatives à la prophylaxie du cancer du sein par l'administration de raloxifène

(30) Priority: 30.10.1996 US 29850 P; 29.11.1996 GB 9624800; 10.03.1997 US 40260 P
(43) Date of publication of application: 06.05.1998
(73) Proprietor: ELI LILLY AND COMPANY, Indianapolis, Indiana 46285 (US)
(72) Inventor: Cohen, Fredric Jay, Indianapolis, Indiana 46236 (US); Glusman, Joan Ellen, Indianapolis, Indiana 46234 (US); Knickerbocker, Ronald Keith, Indianapolis, Indiana 46250 (US); Nickelsen, Nikolaus Thomas, 65812 Bad Soden (DE); Scott, Teri Janine, Carmel, Indiana 46032 (US)
(74) Representative: Denholm, Anna Marie

(56) References cited:
- EP-A- 0 652 004
- EP-A- 0 674 903
- EP-A- 0 680 758
- WO-A-96/22771
- WO-A-97/35571
- US-A- 4 418 068
- "FIRST PHASE III RESULTS WITH RALOXIFENE" DATABASE FILE 129, PHIND; DIALOG INFORMATION SERVICES; AN=540210, 13 June 1997, page 17 XP002054373
- V.C. JORDAN ET AL.: "ALTERNATE ANTIESTROGENS AND APPROACHES TO THE PREVENTION OF BREAST CANCER" JOURNAL OF CELLULAR BIOCHEMISTRY, vol. 58, no. S.22, 1995, pages 51-57, XP002054374
- "MARKET ANALYSIS FROM DECISION RESOURCES: BREAST CANCER" DATABASE FILE 187, FDC REPORTS; DIALOG INFORMATION SERVICES, AN=1249, vol. 1, no. 4, 1 April 1996, XP002054375
- M.A. ANZANO ET AL.: "CHEMOPREVENTION OF MAMMARY CARCINOGENESIS IN THE RAT: COMBINED USE OF RALOXIFENE AND 9-CIS-RETINOIC ACID" JOURNAL OF THE NATIONAL CANCER INSTITUTE, vol. 88, no. 2, January 1996, pages 123-125, XP002054376

## Description

This invention relates to the prophylaxis of breast cancer in women.

Breast adenocarcinoma or cancer is a major medical problem in women, particularly above the age of thirty-five. Currently in the United States, it is estimated that women have a one in eight chance of developing this disease in the course of their lifetime. Breast carcinoma is a major cause of mortality in women, as well as a cause of disability, psychological trauma, and economic loss. A large number of women contracting this disease eventually die from its effects either directly or indirectly from complications.

Raloxifene has the chemical structure: 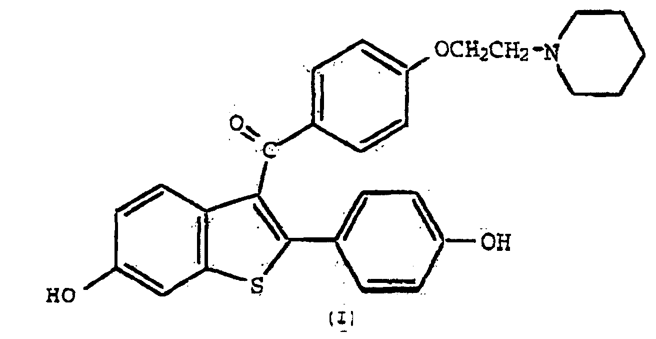 and together with, *inter alia*, its pharmaceutically acceptable salts was first described in U.S. Patent Specification No. 4,418,068.

U.S. Patent No. 4,418,068 teaches that raloxifene is useful in the treatment of mammary tumors and in the prophylaxis of recurring benign mammary fibrocystic disease.

WO 97/35571 discloses raloxifene and pharmaceutically acceptable salts thereof, in particulate form and having a specific size range, and further discloses the use of raloxifene and pharmaceutically acceptable salts thereof for alleviating human pathologies, including osteoporosis, serum lipid lowering and breast cancer.

EP-A-680758 discloses the use of raloxifene and analogues thereof, for minimising the uterotrophic effect of tamoxifen and analogues thereof, when said tamoxifen or analogues thereof is administered to a woman for the treatment or prevention of breast cancer.

Jordan, in J.Cell. Biochem., Supplement 22:51-57 (1995) discusses the role of raloxifene as an antiestrogen. Dialog Information Services, FDC Reports, AN=1249, Pharmaceutical Approval, Monthly, Vol.1, Issue 4, April 1996, mentions potential application of raloxifene in treating osteoporosis and preventing breast cancer and heart attacks. US-A-4418068 discloses raloxifene and ethers and esters thereof, as antiestrogens and antiendrogens. Anzant et. Al., in J. Nat. Cancer. Inst., Vol. 88, Jan 1996, pp 123-125, discusses the chemoprevention of mammary carcinogenesis in the rat, and the combined use of raloxifene and 9-cis-retinoic acid. Unfortunately, clinical trial of raloxifene for the treatment of breast cancer did not support human efficacy. That negative result discouraged further evaluation of raloxifene as a therapeutic agent against breast cancer in humans.

Surprisingly, a chance discovery has now established that raloxifene is highly effective in the prophylaxis of breast cancer. Even more surprisingly, that protective effect has been found to manifest itself particularly strongly in a narrow dosage range.

Thus, according to the invention, there is provided the use of raloxifene, or a pharmaceutically-acceptable salt thereof, for the manufacture of a medicament for the administration of 60 to 120 mg per day of said raloxifene or pharmaceutically acceptable salt thereof, to a post-menopausal woman for inhibiting the development of breast cancer, excluding therapy of existing, clinically detectable breast cancer.

The raloxifene, or pharmaceutically-acceptable salt thereof, should not be administered in an amount greater than 120 mg per day, more preferably not greater than 100 mg per day. Outstanding protective effects have been observed at 60 mg per day, for instance from 50 to 70 mg per day.

The term "inhibiting the development of breast cancer" as used herein is intended to primarily refer to a situation in which *de novo* transformation of normal breast cells to cancerous or malignant cells is inhibited. However, there may be situations in which women have clinically non-detectable cancerous cells in their breasts, and the inhibition of development of such, as yet, clinically insignificant cancers also forms part of the invention. Not included within the scope of this invention, is the therapy of existing, clinically-detectable breast cancer.

Raloxifene, and its pharmaceutically-acceptable salts can be made according to established procedures, such as those described in U.S. Patent Nos. 4,133,814, 4,418,068, and 4,380,635, and U.K. Patent Application 2,293,602. A preferred crystalline form is described in U.K. Patent Application No. 2,293,382 . In general, the synthesis starts with a benzo[b]thiophene having a 6-hydroxyl group and a 2-(4-hydroxyphenyl) group. The hydroxyl groups of the starting compound are protected, the 3-position is acylated, and the product deprotected to form the desired compounds.

Raloxifene forms pharmaceutically-acceptable acid and base addition salts with a wide variety of organic and inorganic acids and bases including the physiologically acceptable salts which are often used in pharmaceutical chemistry. Such salts are also part of this invention. Typical inorganic acids used to form such salts include hydrochloric, hydrobromic, hydroiodic, nitric, sulfuric, phosphoric, hypophosphoric and the like. Salts derived from organic acids, such as aliphatic mono and dicarboxylic acids, phenyl substituted alkanoic acids, hydroxyalkanoic and hydroxyalkandioic acids, aromatic acids, aliphatic and aromatic sulfonic acids, may also be used. Such pharmaceutically acceptable salts thus include acetate, phenylacetate, trifluoroacetate, acrylate, ascorbate, benzoate, chlorobenzoate, dinitrobenzoate, hydroxybenzoate, methoxybenzoate, methylbenzoate, o-acetoxybenzoate, naphthalene-2-benzoate, bromide, isobutyrate, phenylbutyrate, β-hydroxybutyrate, butyne-1,4-dioate, hexyne-1,4-dioate, caprate, caprylate, chloride, cinnamate, citrate, formate, fumarate, glycollate, heptanoate, hippurate, lactate, malate, maleate, hydroxymaleate, malonate, mandelate, mesylate, nicotinate, isonicotinate, nitrate, oxalate, phthalate, teraphthalate, phosphate, monohydrogenphosphate, dihydrogenphosphate, metaphosphate, pyrophosphate, propiolate, propionate, phenylpropionate, salicylate, sebacate, succinate, suberate, sulfate, bisulfate, pyrosulfate, sulfite, bisulfite, sulfonate, benzene-sulfonate, p-bromobenzenesulfonate, chlorobenzenesulfonate, ethanesulfonate, 2-hydroxyethanesulfonate, methanesulfonate, naphthalene-1-sulfonate, naphthalene-2-sulfonate, p-toluenesulfonate, xylenesulfonate, tartarate. The preferred salt for use in the invention is the hydrochloride salt.

The pharmaceutically-acceptable salts generally have enhanced solubility characteristics compared to the compound from which they are derived, and thus are often more amenable to formulation as liquids or emulsions.

Pharmaceutical formulations can be prepared by procedures known in the art. For example, the compounds can be formulated with common excipients, diluents, or carriers, and formed into tablets, capsules, suspensions, powders . Examples of excipients, diluents, and carriers that are suitable for such formulations include the following: fillers and extenders such as starch, sugars, mannitol, and silicic derivatives; binding agents such as carboxymethyl cellulose and other cellulose derivatives, alginates, gelatin, and polyvinyl pyrrolidone; moisturizing agents such as glycerol; disintegrating agents such as calcium carbonate and sodium bicarbonate; agents for retarding dissolution such as paraffin; resorption accelerators such as quaternary ammonium compounds; surface active agents such as cetyl alcohol, glycerol monostearate; adsorptive carriers such as kaolin and bentonite; and lubricants such as talc, calcium and magnesium stearate, and solid polyethyl glycols.

The compounds can also be formulated as elixirs or solutions for convenient oral administration. Whilst parenteral administration, for instance by intramuscular, subcutaneous or intravenous routes, is a possibility, clearly for prophylaxis this is much less desirable than oral administration. Additionally, the compounds are well suited to formulation as sustained release dosage forms . The formulations can be so constituted that they release the active ingredient only or preferably in a particular part of the intestinal tract, possibly over a period of time. The coatings, envelopes, and protective matrices may be made, for example, from polymeric substances or waxes.

Effective daily doses of raloxifene, or pharmaceutically-acceptable salts, will be from 60 to 120 mg/day, with 60 mg/day particularly preferred.

Although one would expect the prophylactic effect provided by the invention to be immediate, statistically significant data, as compared with the general population, is most apparent where the term of administration is at least six months, preferably at least one year, most preferably at least two years. Clearly, the prevention of breast cancer is such a significant enhancement of a woman's quality of life, that very long term, even life long, therapy is advantageous.

As stated above, the preferred route of administration will be the oral route. For such purposes the following oral dosage forms are available.

### Formulations

| Formulation 1: Raloxifene capsule | |
|---|---|
| Ingredient | Quantity (mg/capsule) |
| Raloxifene HCl | 60 |
| Starch, NF | 112 |
| Starch flowable powder | 225.3 |
| Silicone fluid 350 mm²/scentistokes | 1.7 |

### Preferred tablet formulations include the following:

| Formulation 2: | | |
|---|---|---|
| Ingredient | Quantity (mg) | Function |
| Raloxifene HCl | 60.0 | Active |
| Spray Dried Lactose | 30.0 | Soluble Diluent |
| Anhydrous Lactose | 12.0 | Soluble Diluent |
| Povidone | 12.0 | Binder |
| Polysorbate 80 | 2.4 | Wetting Agent |
| Crospovidone | 14.4 | Disintegrant |
| Magnesium Sterate | 1.2 | Lubricant |
| (Core Tablet Weight | 240.0) | |
| Film Coating | | |
| Color Mixture White | 12.0 | Coloring Agent |
| Talc | trace | Polishing Aid |
| Carnauba Wax | -- | Polishing Aid |

| Formulation 3: | | |
|---|---|---|
| Ingredient | Quantity (mg) | Function |
| Raloxifene HCl | 60.0 | Active |
| Spray Dried Lactose | 29.4 | Soluble Diluent |
| Anhydrous Lactose | 120.0 | Soluble Diluent |
| Povidone | 12.0 | Binder |
| Polysorbate 80 | 2.4 | Wetting Agent |
| Crospovidone | 14.4 | Disintegrant |
| Magnesium Sterate | 1.2 | Lubricant |
| (Core Tablet Weight | 240.0) | |
| Film Coating | | |
| Color Mixture White | 12.0 | Coloring Agent |
| Talc | -- | Polishing Aid |
| Carnauba Wax | trace | Polishing Aid |

### TEST PROCEDURE

The results of clinical studies with raloxifene (in the form of the hydrochloride salt) are now presented to illustrate the protective effect of the invention.

The principal design was double-blind randomized, and placebo-controlled in approximately 7500 post-menopausal women. The studies generally had three groups (arms) of patients assigned randomly to placebo, 60 mg of drug per day, or 120 mg of drug per day, enrolled in equal numbers per group, all via the oral route.

Patients selected for these studies were post-menopausal women (2+ years since the last menstrual period) between the ages of approximately fifty to eighty years old. In addition, those women were in generally good health, as appropriate for their age.

Exclusion criteria from participating in this study included past or present cancer. In particular, no woman was allowed to participate in the study who currently had or was suspected to have a history of, breast carcinoma or other estrogen-dependent neoplasia. This exclusion criterion generated a population of patients which reflected that of the general population in regard to the potential for developing breast cancer.

Potential patients were screened prior to enrollment into the study. Patients were required to reveal current medical conditions and histories. All potential patients were required to have either a baseline mammogram or breast ultrasound evaluation - to screen for the presence of breast cancer. If there was some reason for a patient to leave the study, and breast cancer was or might be expected, this patient would receive a breast cancer evaluation. The diagnosis of breast cancer was made histopathologically from biopsy or surgical specimens.

The results shown in Table I are for patients who were found through various diagnostic methods, including the one year mammogram results, to have breast adenocarcinoma, or cancer. These patients were immediately discontinued from study participation and their status unblinded to reveal the therapy (arm) to which they had been randomized.

The number of patients in each arm of the study was 5078 on the combined two dosage levels of raloxifene and 2539 on placebo.

**Table I**

| Patient No. | Treatment Group | No.of Months |
|---|---|---|
| 4053 | Placebo | 5 |
| 3057 | Placebo | 12 |
| 6637 | Placebo | 12 |
| 2233 | Placebo | 1 |
| 2652 | Placebo | 4 |
| 7666 | Placebo | 3.5 |
| 3994 | Placebo | 2 |
| 4678 | Placebo | 6 |
| 6018 | Placebo | 15 |
| 3817 | Placebo | 13 |
| 1858 | Placebo | 13 |
| 0238 | Placebo | 3 |
| 3930 | 60 mg Raloxifene | 11 |
| 2693 | 120 mg Raloxifene | 4 |
| 5801 | 120 mg Raloxifene | 4 |
| 0393 | 120 mg Raloxifene | 4 |
| 0032 | 120 mg Raloxifene | 12 |

The results of the data indicate that the relative risk for the patients in this study of being diagnosed as having breast cancer was 1.0 (by definition) for placebo and 0.21 (95% confidence interval, 0.07 to 0.59) for those taking raloxifene (data on patients for both the 60 mg and 120 mg doses were pooled), as compared with placebo. The 60 mg dose was more favorable and is therefore a preferred embodiment of the current invention.

When all pertinent studies on raloxifene were combined, a total of 14 placebo-arm patients incurred breast cancer, while a total of 9 raloxifene patients incurred breast cancer. The total number of enrolled placebo patients was 3087, while the total number of enrolled raloxifene patients was 6584.

The following Table II reports some further results from a study utilizing different raloxifene dosage levels.

**Table II**

| Patient No. | Treatment Group | No.of Months |
|---|---|---|
| 0888 | Placebo | 22 |
| 3601 | Placebo | 21 |
| 2889 | Raloxifene 150 mg | 20 |
| 3857 | Raloxifene 30 mg | 13 |
| 3622 | Raloxifene 30 mg | 8 |
| 5415 | Raloxifene 60 mg | 13 |

For all studies pooled, the crude relative risk estimate (RR) of developing breast carcinoma for raloxifene vs. placebo was 0.31(95% confidence interval, 0.13 to 0.71). The probability that these treatment group differences in reported incidence were due to random chance is approximately 0.6%. Approximate incidence rates of cancer reports for each arm for raloxifene and placebo were: Placebo = 4.23, Raloxifene 30 mg = 4.0, Raloxifene 60 mg = 0.6, Raloxifene 120 mg = 1.6, Raloxifene 150 mg = 2.6 (Incidence rates are presented as number of case reports per 1,000 patient-years of exposure to drug).

## Claims

1. The use of raloxifene, or a pharmaceutically acceptable salt thereof, for the manufacture of a medicament for the administration of 60 to 120 mg per day of said raloxifene or pharmaceutically acceptable salt thereof, to a post-menopausal woman for inhibiting the development of breast cancer, excluding therapy of existing, clinically detectable breast cancer.

2. The use as claimed in Claim 1 for the manufacture of a medicament for the administration of 60 mg per day of said raloxifene or salt thereof.

## Patentansprüche

1. Verwendung von Raloxifen oder eines pharmazeutisch annehmbaren Salzes hiervon zur Herstellung eines Arzneimittels zur Verabreichung von 60 bis 120 mg pro Tag des Raloxifens oder eines pharmazeutisch annehmbaren Salzes hiervon an eine postmenopausale Frau zur Hemmung der Entwicklung von Brustkrebs, einschließlich der Therapie von existiereridem, klinisch detektierbarem Brustkrebs.

2. Verwendung nach Anspruch 1 zur Herstellung eines Arzneimittels zur Verabreichung von 60 mg pro Tag an Raloxifen oder einem Salz hiervon.

## Revendications

1. Utilisation de raloxifène ou d'un sel pharmaceutiquement acceptable de celui-ci pour la préparation d'un médicament destiné à l'administration de 60 à 120 mg par jour dudit raloxifène ou sel pharmaceutiquement acceptable de celui-ci à des femmes post-ménopausiques pour inhiber le développement du cancer du sein, à l'exclusion de la thérapie d'un cancer du sein existant, cliniquement décelable.

2. Utilisation telle que revendiquée dans la revendication 1 pour la préparation d'un médicament destiné à l'administration de 60 mg par jour dudit raloxifène ou sel de celui-ci.
